(19) Europäisches Patentamt
European Patent Office
Office européen des brevets

(11) Publication number: 0 160 544
A2

(12) EUROPEAN PATENT APPLICATION

(21) Application number: 85302982.5

(22) Date of filing: 26.04.85

(51) Int. Cl.[4]: C 11 C 3/14
C 07 C 51/353, C 07 C 67/33-3

(30) Priority: 26.04.84 GB 8410625

(43) Date of publication of application:
06.11.85 Bulletin 85/45

(84) Designated Contracting States:
BE CH DE FR GB IT LI NL

(71) Applicant: INDIAN EXPLOSIVES LIMITED
ICI House 34 Chowringhee Road
Calcutta-700 071 West Bengal(IN)

(72) Inventor: Basu, Amitabha Alchemie Research Centre
Private Limited CAFI Site PO Box 155 Belapur Road
Thane 400 601 Maharashtra(IN)

(72) Inventor: Bhaduri, Sumit Alchemie Research Centre
Private Limited CAFI Site PO Box 155 Belapur Road
Thane 400 601 Maharashtra(IN)

(72) Inventor: Kasar, T.G.K. Alchemie Research Centre
Private Limited CAFI Site PO Box 155 Belapur Road
Thane 400 601 Maharashtra(IN)

(74) Representative: Collier, Jeremy Austin Grey et al,
J.A.Kemp & Co. 14, South Square Gray's Inn
London WC1R 5EU(GB)

(54) **Process for the manufacture of conjugated polyenoic fatty compounds.**

(57) Non-conjugated fatty compounds, especially natural glycerides, are isomerized to the corresponding conjugated compounds by contact with a cationic complex of a Group VIII transition metal such as rhodium or iridium.

EP 0 160 544 A2

Croydon Printing Company Ltd

# PROCESS FOR THE MANUFACTURE OF CONJUGATED POLYENOIC FATTY COMPOUNDS

The present invention relates to the manufacture of conjugated polyenoic (including dienoic) fatty compounds from corresponding non-conjugated fatty compounds.

Naturally occurring triglycerides (fats and fatty oils), the free fatty acids of such fats and oils and the alkyl esters of these fatty acids contain non-conjugated fatty compounds. The conjugated fatty acids or esters obtained from such non-conjugated fatty compounds are useful in paints, varnishes, coatings and printing inks to which they impart improved properties, e.g. improved drying properties.

Most hitherto reported isomerisation processes have been applied to the methyl esters of fatty acids derived from natural oils, e.g. methyl linoleate. However, very few of them work with natural poly-unsaturated fats and oils.

U.S. Patents 3,373,175 and 3,392,177 propose a method for the isolation of a conjugated drying oil by reaction of a vegetable oil with an excess of iron pentacarbonyl for 2-4 hours at 185°C, removal of the complex of the conjugated acid formed, and its decomposition with ferric chloride or carbon monoxide under pressure. However, stoichiometric amounts of iron pentacarbonyl are

required in this process.

In British Patent 1,408,189, French Patent 2,159,402 and Swiss Patent 578,044, the use of alcoholates of alkali and alkaline earth metals in a very polar aprotic solvent is proposed. The isomerization is carried out at temperature up to 160°C in the presence of 1-5% of the methylate or butylate of the metal in a solvent such as dimethyl-sulphoxide, dimethylformamide, dimethylacetamide, or N-methyl pyrrolidine.

British Patent 590784 describes a catalytic process for conjugation of double bonds in fatty substances in the vapor phase at high temperatures. The catalysts used are amorphous silica, activated fulling clay and the silicates and oxides of all metals except the alkali and alkaline earth metals.

German Patent 2,049,937 describes a process for catalytic conjugation of double bonds in polyunsaturated higher carboxylic acids in the form of their esters with lower monovalent alcohols or the triglycerides but especially in the form of free carboxylic acids. The catalysts used are complexes of rhodium or ruthenium in combination with metallic chlorides or bromides.

French Patent 81430013.3 describes a process for catalytic conjugation of polyunsaturated fatty compounds in the liquid phase with ruthenium on charcoal at a temperature of 180-250°C in the presence of a protonic compound.

The drawbacks of the prior art processes reside essentially in the extreme conditions of reaction and the impossibility of recovering the catalyst quantitatively at the end of the reaction without substantial loss in its activity. The present invention reduces or avoids this problem.

Accordingly, the present invention provides a process for the manufacture of a conjugated polyenoic fatty compound from a corresponding non-conjugated fatty compound which comprises contacting a non-conjugated polyunsaturated triglyceride, fatty acid or fatty acid ester with, as catalyst, a cationic complex of a transition metal of Group VIII in the presence of a suitable solvent to produce the desired corresponding conjugated polyenoic fatty compound.

The preferred metals of Group VIII are rhodium and iridium. The advantage of using rhodium and iridium catalysts over other homogeneous catalysts lies in the fact that it is possible to remove them at the end of the reaction by solvent extraction.

Although it has been reported by Shrock, R. R. and J. A. Osborn, J. Amer. Chem. Soc. 98, 2134 (1976); ibid; 98,2113 (1976); ibid; 98,4450 (1976) that cationic Rhodium I complexes of the type (ligand) $RhL_2$ wherein L = $PPh_3$, 1,2-bisdiphenyl phosphinoethine, etc. are very active hydrogenation catalysts for fatty oils, their use in the present invention as conjugation catalysts is suprising because no attempt has ever been made before to employ them

for this purpose. Likewise, Rhodium III, Iridium I and Iridium III complexes (wherein the suffixes I and III refer to the valencies of the metals) may also be employed as the cationic catalytic complexes in the process of the present invention.

The catalytic complexes may be synthesised in a separate preparation step and then employed in the process of the present invention, or they may be generated in situ in the course of the action.

According to a preferred embodiment of the invention a non-conjugated polyunsaturated natural oil or the free fatty acid thereof or an alkyl ester of the acid, is heated, and normally refluxed, with a cationic catalyst complex of rhodium or iridium in an appropriate solvent, preferably in an atmosphere of inert gas. The isomerisation reaction is usually effected at temperatures of 50°C to 160°C and at pressures from 1 to 10 atmospheres. The reaction time required can be gauged by taking periodic gas chromatographic samples of the product mixture and this reaction time varies depending on the specific catalyst used, the temperature of the reaction and the solvent employed.

The solvent for the reaction is preferably a hydroxylic solvent which is an alcohol such as methanol, ethanol, isopropyl alcohol, butanol or cyclohexanol, a chlorinated hydrocarbon solvent such as chloroform, dichloromethane or chlorobenzene, or a polar inert solvent

such as acetone or acetonitrile, or mixtures of any of these.

The non-conjugated triglycerides that may be isomerized by the process of the present invention include linseed oil, safflower oil, china wood oil, dehydrated castor oil, soyabean oil, grape seed oil, sunflower oil, rapeseed oil and cotton seed oil. Also employable are esters of the free acids of such triglycerides with lower aliphatic alcohols.

Alternatively, the process may be applied to the free fatty acids of the fats and oils referred to. Examples of these acids are the polyunsaturated higher carboxylic acids such as linoleic acid, linolenic acid, linolaidic acid, and linolaidenic acid. These carboxylic acids may be employed in admixture with one another or with other saturated or mono-olefinic higher carboxylic acids, e.g. fatty acids of natural origin such as linseed oil acids, safflower oil acids, soyabean oil acids, rapeseed oil acids, cottonseed oil acids or sunflower oil acids.

Alternatively, the polyunsaturated higher carboxylic acids may be used in the form of their esters with lower monovalent alcohols.

The catalytic complexes preferably employed in the process of the present invention can be dimeric complexes represented by one or other of the formulae:

$[(\text{Diene})\text{MCl}]_2$ or $[(\text{Diene})\text{M}(\text{acac})]_2$

wherein : M = rhodium or iridium;

Diene = 1,5-cyclooctadiene, norbornadiene (NBD), 1,3-butadiene or 1,3-cyclohexadiene; and

acac = acetyl acetone.

In addition to the above mentioned dimeric complexes, other specific catalytic complexes which may be employed are identified hereafter by the respective formulae I to VIII.

$$[M(Diene)\ L_2]^+ A^- \qquad \text{Formula I}$$

wherein : M = rhodium or iridium;

Diene = 1,5-cyclo octadiene; norbornadiene; 1,3-cyclohexadiene or 1,3-butadiene;

A = $ClO_4$, $BPh_4$ or $PF_6$; and

L = $P(C_6H_5)_3$, $P(C_6H_5)_2CH_3$, $P(C_6H_5)(CH_3)_2$, $P(C_6H_5)_2(C_6H_{11})$, $P(OC_6H_5)_3$, $P(C_6H_5)_2OCH_3$, $As(C_6H_5)_3$ or $NH_2C(CH_3)_3$ or

$L_2$ = 1,2-bis diphenyl phosphino ethane (diphos); 1-diphenyl arsino-2-diphenyl phosphino ethane; 2,2'-bipyridine or 1,10-phenanthroline.

$$[ML_2H_2S_2]^+ \ A^- \qquad \text{Formula II}$$

wherein : M = rhodium or iridium;

A = $ClO_4$, $BPh_4$ or $PF_6$;

L = $P(C_6H_5)_3$, $P(C_6H_5)_2(C_6H_{11})$, $P(C_6H_5)(CH_3)_2$, $P(C_6H_5)_2CH_3$ or $As(C_6H_5)_3$; and

S = acetone, ethanol, tetrahydrofuran, acetonitrile.

$[Ir(Diene)H_2L_2]^+ A^-$ Formula III

wherein : Diene = 1,5-cyclooctadiene; norbornadiene, 1,3-cyclohexadiene or 1,3-butadiene;

A = $ClO_4$, $BPh_4$ or $PF_6$ and

L = $P(C_6H_5)_3$, $P(C_6H_5)_2CH_3$ or $P(C_6H_5)(CH_3)_2$ or

$L_2$ = 1,2-diphenyl phosphino ethane.

$[Ir(Diene)LPy]^+ A^-$ Formula IV

wherein : Diene = 1,5-cyclooctadiene, norbornadiene; 1,3-cyclohexadiene or 1,3-butadiene;

Py = pyridine;

A = $ClO_4$, $BPh_4$ or $PF_6$; and

L = $P(C_6H_5)_3$, $P(CH(CH_3)_2)_3$, $P(C_6H_{11})_3$, $P(C_6H_5)_2CH_3$; $P(C_6H_5)(CH_3)_2$ and $P(C_6H_5)_2(C_6H_{11})$.

$[Rh(L_1)_2(L_2)_2H_n]^+ A^-$ Formula V

wherein : A = $ClO_4$, $BPh_4$ or $PF_6$ ; and

when n = O:

$L_1 = L_2$ = $P(C_6H_5)_2CH_3$, $P(C_6H_5)(CH_3)_2$ or $P(OCH_3)_2(C_6H_5)$

when n = 2:

$L_1$ = $P(C_6H_5)(CH_3)_2$, $P(C_6H_5)_2CH_3$, $P(C_6H_5)_3$, $P(C_6H_5)_2(C_6H_{11})$ or As $(C_6H_5)_3$ when

$L_2 = As(C_6H_5)(CH_3)_2$ or

$(L_2)_2$ = 2,2'-bipyridine or

$L_1 = L_2$ = $P(C_6H_5)(CH_3)_2$; $P(CH_3)_3$ or As $(C_6H_5)(CH_3)_2$.

$$[\text{Rh (Diene) } L_1 (L_2)_2]^+ \ A^- \qquad \text{Formula VI}$$

wherein : Diene = norbornadiene;

A = $ClO_4$, $BPh_4$ or $PF_6$ and

$L_1 = L_2$ = $P(CH_3)_3$, $P(CH_3)_2(C_6H_5)$ or $As(CH_3)_2(C_6H_5)$ or

$L_1$ = $P(CH_3)_2C_6H_5$ or As $(CH_3)_2(C_6H_5)$ when $(L_2)_2$ = 1,2-diphenyl phosphino ethane or 1-diphenyl arsino-2-diphenyl phosphino ethane.

$$[\text{Rh (Diene) } L_2CO]^+ \ A^- \qquad \text{Formula VII}$$

wherein : Diene = norbornadiene; 1,3-butadiene or 1,5-cyclooctadiene;

A = $ClO_4$, $BPh_4$ or $PF_6$ and

L = $P(C_6H_5)_3$ or $P(C_6H_5)_2CH_3$.

$$[\text{Rh } L_x(CO)_yS_z]^+ \ A^- \qquad \text{Formula VIII}$$

wherein : A = $ClO_4$, $BPh_4$ or $PF_6$;

S = solvent molecule like dimethyl formamide, dimethyl acetamide, acetone or acetonitrile;

L = $P(C_6H_5)_3$ or $P(C_6H_5)_2\ CH_3$ and

x = 2, y = 2, z = 0 or

x = 2, y = 1, z = 1 or

x = 3, y = 1, z = 0 or

X = 3, y = 2, z = 0

The complexes identified by Formulae II to VIII above can be synthesised separately by using known methods such as those described by Schrock, R. R. and Osborn, J.A., Journal of the American Chemical Society, 93,2397(1971), and then employed as catalysts in the process of the present invention. Alternatively, they may be generated in situ in the reaction mixture from complexes of the Formula I by treatment with appropriate reagents.

If desired, the catalytic complexes may be heterogenised by incorporating the phosphine ligand into a polymeric matrix such as polystyrene. Since the complexes are cationic in nature, it is also possible to anchor these catalysts on cation exchange resins or zeolites which act as substrates. However, the effectiveness of the complexes as catalysts partly depends on the solubility of the substrate and the catalyst in the solvent used.

At the end of the reaction, the catalyst can be recovered quantitatively by simple solvent extraction of the substrate. For this purpose, a non-polar solvent such as pentane or hexane can be employed. The polar nature of the catalyst causes it to precipitate out on addition of the solvent and the catalyst can then be recovered in any known way and recycled for use again without any significant

loss of its catalytic properties.

The invention is described in greater detail in the following illustrative Examples.

EXAMPLE 1

Safflower oil (10 parts) was contacted with Rh(NBD) $(P(C_6H_5)_3)_2$ $ClO_4$ (0.1 part) in a solvent mixture of acetone and methanol at 70°C under a $N_2$ atmosphere for 12 hours. At the end of the reaction, the catalyst was isolated by solvent extraction with methanol. The isomerised oil which was separated was converted to its methyl ester by reaction with sodium methoxide. Analysis of this methyl ester by gas liquid chromatography showed 90% conversion of the linoleate present in the starting oil to its conjugated isomer.

EXAMPLE 2

In a 100 ml flask fitted with a reflux condenser, 100 parts of soyabean acid methyl ester (54% linoleic acid methyl ester; 28% oleic acid methyl ester; 5% Linolenic acid methyl ester, 8% palmitic acid methyl ester; 4% stearic acid methyl ester and the remaining $C_{14}$ to $C_{20}$ acid methyl ester) were refluxed with 1 part of $[Rh(NBD)(P(C_6H_5)_3)_2]ClO_4$ in a 90:10 solvent mixture of acetone and methanol under a $N_2$ atmosphere for 6 hours. At the end of the reaction, the solvent was removed under vacuum and the residue washed with pentane to remove the catalyst with precipitates. The mixture of fatty acid esters thus separated was analysed by gas-liquid

chromatography (glc) and showed 90% conjugation of the methyl linoleate present in the initial mixture.

EXAMPLE 3

In a 100 ml flask fitted with a reflux condenser, 100 parts of safflower oil (composition 77% linoleic acid, 14.5% oleic acid, 5.6% palmitic acid, 2.1% stearic acid, remaining $C_{14}$ to $C_{20}$ saturated acids) were refluxed with 1 part of $[Rh(NBD)(P(C_6H_5)_3)_2]ClO_4$ in a 90:10 solvent mixture of acetone and methanol under a $N_2$ atmosphere for 12 hours. At the end of the reaction, the solvent was removed and the catalyst precipitated from the oil by addition of pentane. The catalyst was isolated by filtration and isomerised oil was obtained by removing pentane from the filtrate. The isomerised oil was analysed by glc after conversion to its methyl ester.

Preparation of methyl ester

1 ml of isomerised oil was dissolved in 10 ml of dry methanol and 3 mg of sodium was added. The solution was refluxed under $N_2$ for 30 minutes. The solution at the end of the reaction was homogeneous and on analysis by glc showed 90% conversion of the linoleate present in the starting oil to its conjugated isomer.

The catalyst isolated from the above reaction was mixed again with 100 parts of safflower oil in a 90:10 acetone-methanol solvent mixture and refluxed under $N_2$ for 12 hours. The isomerised oil that was isolated at the end of the reaction by the same procedure as above showed 87% conjugation of the linoleate present in the starting oil

on analysis of its methyl ester by glc.

The catalyst was recycled once again exactly as above and yielded an isomerised oil with 82% conjugation of starting linoleate.

EXAMPLE 4

100 parts of safflower acid (composition conforming to safflower oil used in example 3) were refluxed with 1 part of [ $Rh(EtOH)_2(PPh_3)_2H_2$ ]$ClO_4$ in a 80:20 $CHCl_3$-EtOH mixture under $N_2$ for 12 hours. At the end of the reaction, the catalyst was isolated as in the previous example. The isomerised acid was converted to its methyl ester by refluxing with 0.5% $H_2SO_4$ in $CH_3OH$ and analysed by glc which indicated 70% conjugation of the linoleic acid present in the starting mixture.

EXAMPLE 5

100 parts of Linseed oil (composition 51% Linolenic acid, 17% linoleic acid, 22 % oleic acid, the remaining saturated $C_{16}$ - $C_{24}$ acids) were refluxed with 1 part of [ $Ir(NBD)(PPh_3)_2$ ]$ClO_4$ in a 90:10 $CH_2Cl_2$-MeOH mixture under $N_2$ for 12 hours. At the end of the reaction, the catalyst was isolated as in Example 2. The isomerised oil after conversion to its methyl ester as in Example 2 showed 52% of conjugated dienes and 12% conjugated trienes on glc analysis.

EXAMPLE 6

100 parts of linseed oil fatty acid (with the same composition as the linseed oil of Example 5) were refluxed

with 1 part of [Rh(NBD)(Diphos)]$ClO_4$ in a 90:10 $CH_2Cl_2$-MeOH mixture for 12 hours under $N_2$. The product mixture after removal of catalyst was converted to the methyl ester with 0.1% $H_2SO_4$ in $CH_3OH$. GLC analysis showed 56% of conjugated diene and 6% conjugated triene.

EXAMPLE 7

Safflower oil methyl ester (having the same composition as the safflower oil of Example 3) was isomerised with [Rh $H_2(PPh_2cy)_2$ (acetone)$_2$]$PF_6$ that was generated in situ. For this purpose, 1 part of [RH$(PPh_2cy)_2$(NBD)] $PF_6$ (cy = cyclohenyl, $C_6H_{11}$ and Ph = $C_6H_5$) in acetone was first treated with $H_2$ for 10 minutes at room temperature. At the end of this period, $H_2$ was replaced by $N_2$, 100 parts of safflower oil methyl ester were added and the mixture refluxed under $N_2$ for 5 hours. GLC analysis at the end of the reaction after isolation of the catalyst indicated 90% conjugation of the methyl linoleate present in the starting mixture.

By the process of the present invention, it is possible to bring about isomerisation within a carbon chain to form a conjugated structure. The process possesses the particular advantage in that by employing the catalyst system described, it is possible to conjugate directly naturally occuring triglycerides. Earlier catalytic systems were known to catalyse the isomerisation of fatty acid esters of lower aliphatic alcohols but failed in attempts to isomerise natural triglycerides. Over and above

this, the process also possesses the added advantage of making it possible readily to recover the catalyst from the reaction mixture at the end of the isomerisation reaction.

CLAIMS

1. A process for the manufacture of a conjugated polyenoic fatty compound from a corresponding non-conjugated fatty compound which comprises contacting a non-conjugated polyunsaturated triglyceride, fatty acid or fatty acid ester with, as catalyst, a cationic complex of a transition metal of Group VIII in the presence of a solvent.

2. A process as claimed in claim 1 wherein the said transition metal of Group VIII is rhodium or iridium.

3. A process as claimed in claim 2 wherein the said catalytic complex is a dimeric complex of the formula:

$$[(Diene)MCl]_2 \quad \text{or} \quad [(Diene)M(acac)]_2$$

wherein: M = rhodium or iridium;

Diene = 1,5-cyclooctadiene, norbornadiene, 1,3-butadiene or 1,3-cyclohexadiene; and

acac = acetyl acetone.

4. A process as claimed in claim 2 wherein the said catalytic complex has one of the following Formulae I to VIII :

$$[M(Diene)\ L_2]^+A^- \qquad \text{Formula I}$$

wherein : M = rhodium or iridium;

Diene = 1,5-cyclo octadiene; norbornadiene; 1,3-cyclohexadiene or 1,3-butadiene;

A = $ClO_4$, $BPh_4$ or $PF_6$; and

L = $P(C_6H_5)_3$, $P(C_6H_5)_2CH_3$, $P(C_6H_5)(CH_3)_2$, $P(C_6H_5)_2(C_6H_{11})$, $P(OC_6H_5)_3$, $P(C_6H_5)_2OCH_3$,

$As(C_6H_5)_3$ or $NH_2C(CH_3)_3$ or

$L_2$ = 1,2-bis diphenyl phosphino ethane (diphos); 1-diphenyl arsino-2-diphenyl phosphino ethane; 2,2'-bipyridine or 1,10-phenanthroline.

$[ML_2H_2S_2]^+ \quad A^-$ Formula II

wherein : M = rhodium or iridium;

A = $ClO_4$, $BPh_4$ or $PF_6$;

L = $P(C_6H_5)_3$, $P(C_6H_5)_2(C_6H_{11})$, $P(C_6H_5)(CH_3)_2$, $P(C_6H_5)_2CH_3$ or $As(C_6H_5)_3$; and

S = acetone, ethanol, tetrahydrofuran, acetonitrile.

$[Ir\ (Diene)\ H_2L_2]^+ \quad A^-$ Formula III

wherein : Diene = 1,5-cyclooctadiene; norbornadiene, 1,3-cyclohexadiene or 1,3-butadiene;

A = $ClO_4$, $BPh_4$ or $PF_6$ and

L = $P(C_6H_5)_3$, $P(C_6H_5)_2CH_3$ or $P(C_6H_5)(CH_3)_2$ or

$L_2$ = 1,2-diphenyl phosphino ethane.

$[Ir\ (Diene)\ LPy]^+ \quad A^-$ Formula IV

wherein : Diene = 1,5-cyclooctadiene, norbornadiene; 1,3-cyclohexadiene or 1,3-butadiene;

Py = pyridine;

A = $ClO_4$, $BPh_4$ or $PF_6$; and

L = $P(C_6H_5)_3$, $P(CH(CH_3)_2)_3$, $P(C_6H_{11})_3$, $P(C_6H_5)_2CH_3$; $P(C_6H_5)(CH_3)_2$ and $P(C_6H_5)_2(C_6H_{11})_3$.

$[Rh(L_1)_2(L_2)_2H_n]^+ A^-$ Formula V

wherein : A = $ClO_4$, $BPh_4$ or $PF_6$ ; and

when n = 0:

$L_1 = L_2$ = $P(C_6H_5)_2CH_3$, $P(C_6H_5)(CH_3)_2$ or $P(OCH_3)_2(C_6H_5)$

when n = 2:

$L_1$ = $P(C_6H_5)(CH_3)_2$, $P(C_6H_5)_2CH_3$, $P(C_6H_5)_3$, $P(C_6H_5)_2(C_6H_{11})$ or As $(C_6H_5)_3$ when

$L_2$ = As $(C_6H_5)(CH_3)_2$ or

$(L_2)_2$ = 2,2'-bipyridine or

$L_1 = L_2$ = $P(C_6H_5)(CH_3)_2$; $P(CH_3)_3$ or $As(C_6H_5)(CH_3)_2$.

$[Rh\ (Diene)\ L_1(L_2)_2]^+ A^-$ Formula VI

wherein : Diene = norbornadiene;

A = $ClO_4$, $BPh_4$ or $PF_6$ and

$L_1 = L_2$ = $P(CH_3)_3$, $P(CH_3)_2(C_6H_5)$ or $As(CH_3)_2(C_6H_5)$ or

$L_1$ = $P(CH_3)_2C_6H_5$ or As $(CH_3)_2(C_6H_5)$ when $(L_2)_2$ = 1,2-diphenyl phosphino ethane or 1-diphenyl arsino-2-diphenyl phosphino ethane.

$[Rh\ (Diene)\ L_2CO]^+ A^-$ Formula VII

wherein : Diene = norbornadiene; 1,3-butadiene or 1,5-cyclooctadiene;

A = $ClO_4$, $BPh_4$ or $PF_6$ and

L = $P(C_6H_5)_3$ or $P(C_6H_5)_2CH_3$.

$$[Rh\ L_x(CO)_yS_z]^+\ A^-$$ Formula VIII

wherein : A = $ClO_4$, $BPh_4$ or $PF_6$;

S = solvent molecule like dimethyl formamide, dimethyl acetamide, acetone or acetonitrile;

L = $P(C_6H_5)_3$ or $P(C_6H_5)_2\ CH_3$ and

x = 2, y = 2, z = 0 or

x = 2, y = 1, z = 1 or

x = 3, y = 1, z = 0 or

X = 3, y = 2, z = 0

5. A process as claimed in any of claims 1 to 4 wherein a non-conjugated polyunsaturated triglyceride or natural oil or the free fatty acid thereof or an alkyl ester of said acid is refluxed with the said catalyst complex in the said solvent.

6. Process according to any of claims 1 to 4 wherein the reaction is effected at a temperature of from 50°C to 150°C and a pressure of from 1 to 10 atmospheres.

7. A process as claimed in any of claims 1 to 6 wherein the reaction is effected in an atmosphere of inert gas.

8. A process as claimed in any of claims 1 to 7 wherein the said solvent is an alcohol, a chlorinated hydrocarbon solvent, a polar, inert solvent or a mixture of

any of these.

9. Process according to claim 8 wherein the said solvent is methanol, ethanol, isopropyl alcohol, butanol, cyclohexanol, chloroform, dichloromethane, chlorobenzene, acetone, acetonitrile, of a mixture of any of these.

10. A process as claimed in any of claims 1 to 9 wherein the non-conjugated fatty compound is linseed oil, safflower oil, china wood oil, dehydrated castor oil soyabean oil, grapeseed oil, sunflower oil, rapeseed oil, cottonseed oil, linseed oil acids, safflower oil acids, soyabean oil acids, rapeseed oil acids, cottonseed oil acids, sunflower oil acids, linoleic acid, linolenic acid, linolaidic acid, linolaidenic acid, an ester of a said fatty acid with a lower aliphatic alcohol or a mixture thereof.

11. Process according to any of claims 1 to 10 wherein the said catalyst complex is formed in situ.

12. Process according to any of claims 1 to 11 wherein the said catalyst complex is provided on a substrate.

13. A process as claimed in any of claims 1 to 12 wherein after the conjugation reaction is complete, the catalyst is recovered by solvent extraction.

14. A process as claimed in claim 13 wherein the solvent used for recovery of the catalyst is a non-polar solvent.